(19) Europäisches Patentamt
European Patent Office
Office européen des brevets

(11) Veröffentlichungsnummer: **0 273 864**
**A2**

(12) # EUROPÄISCHE PATENTANMELDUNG

(21) Anmeldenummer: **87810756.4**

(22) Anmeldetag: **16.12.87**

(51) Int. Cl.[4]: **C 07 C 39/16**
C 07 C 39/17, C 08 K 5/13

(30) Priorität: **22.12.86 CH 5137/86**

(43) Veröffentlichungstag der Anmeldung:
**06.07.88 Patentblatt 88/27**

(84) Benannte Vertragsstaaten:
**BE DE ES FR GB IT NL SE**

(71) Anmelder: **CIBA-GEIGY AG**
**Klybeckstrasse 141**
**CH-4002 Basel (CH)**

(72) Erfinder: **Pitteloud, Rita, Dr.**
**Sur le Village**
**CH-1724 Praroman (CH)**

**Dubs, Paul, Dr.**
**Route des Pralettes 25**
**CH-1723 Marly (CH)**

(54) **Diphenylmethane.**

(57) Verbindungen der Formel I,

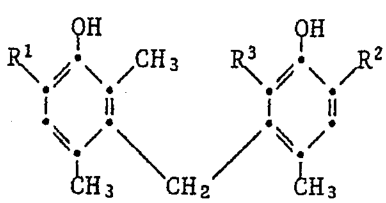

(I)

worin $R^1$ und $R^2$ unabhängig voneinander Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl sind und $R^3$ Methyl oder Ethyl bedeutet, mit der Bedingung, dass die Verbindung 3,3'-Dihydroxy-2,2',4,4',6,6'-hexamethyl-diphenylmethan ausgeschlossen ist, eignen sich zum Stabilisieren von organischem Material gegen thermischen, oxidativen und/oder aktinischen Abbau.

EP 0 273 864 A2

**Beschreibung**

Diphenylmethane

Die vorliegende Erfindung betrifft neue Bis[3-hydroxyphenyl]methane, deren Verwendung zum Stabilisieren von organischem Material und das mit deren Hilfe gegen thermischen, oxidativen und/oder aktinischen Abbau stabilisierte organische Material.

Aus der US-A-4 507 420 sind Diphenylmethanderivate und deren Verwendung zum Stabilisieren von organischem Material bekannt. In der DE-A-1 051 864 und in einem Artikel von S.R. Finn und J.W.G. Musty in "J. Soc. Chem. Ind. Suppl. Issue No. 1, $S_3$-$S_7$ (1950)" wird die Herstellung von Diphenylmethanderivaten beschrieben.

Gegenstand der vorliegenden Erfindung sind Verbindungen der Formel I,

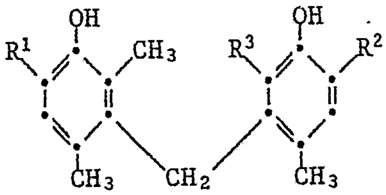
(I)

worin $R^1$ und $R^2$ unabhängig voneinander Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl sind und $R^3$ Methyl oder Ethyl bedeutet, mit der Bedingung, dass die Verbindung 3,3′-Dihydroxy-2,2′,4,4′,6,6′-hexamethyldiphenylmethan ausgeschlossen ist.

$R^1$ und $R^2$ bedeuten als $C_5$-$C_{20}$-Alkyl, welches am α-C-Atom verzweigt ist, zum Beispiel Pentan-2-yl, α-Methylhexyl oder α-Methyldecyl.

$R^1$ und $R^2$ bedeuten als $C_5$-$C_8$-Cycloalkyl, welches gegebenenfalls durch $C_1$-$C_4$-Alkyl substituiert sein kann, zum Beispiel Cyclopentyl, Cyclohexyl, Cyclooctyl oder α-Methylcyclohexyl. Cyclohexyl ist besonders bevorzugt.

$R^3$ ist bevorzugt Methyl und die Reste $R^1$ und $R^2$ sind bevorzugt gleich.

Bevorzugt sind Verbindungen der Formel I, worin $R^1$ und $R^2$ unabhängig voneinander Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl sind und $R_1$ zusätzlich Methyl bedeutet.

Ebenfalls bevorzugt sind Verbindungen der Formel I, worin $R^1$ und $R^2$ unabhängig voneinander Ethyl, Isopropyl oder $C_5$-$C_8$-Cycloalkyl, insbesondere Ethyl, Isopropyl oder Cyclohexyl sind.

Von Interesse sind auch Verbindungen der Formel I, worin $R^1$ Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl ist, $R^2$ Methyl bedeutet und $R^3$ Ethyl darstellt.

Beispiele für Verbindungen der vorliegenden Erfindung sind:
3,3′-Dihydroxy-4,4′-dicyclohexyl-2,2′,6,6′-tetramethyl-diphenylmethan,
3,3′-Dihydroxy-4,4′-diisopropyl,2,2′,6,6′-tetramethyl-diphenylmethan, 3,3′-Dihydroxy-4,4′-diethyl-2,2′,6,6′-tetramethyl-diphenylmethan, 3,3′-Dihydroxy-4,2′-diethyl,2,4′,6,6′-tetramethyl-diphenylmethan.

Die Verbindungen dieser Erfindung bzw. deren Gemische eignen sich zum Stabilisieren von organischen Materialien gegen thermischen, oxidativen und/oder aktinischen Abbau. Beispiele für derartige Materialien sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymre, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinyliden chlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymer, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren Polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Ein weiterer Gegenstand dieser Erfindung sind daher Zusammensetzungen, die organisches Material und mindestens eine Verbindung der Formel I enthalten.

Bei dem organischen Material handelt es sich vorzugsweise um synthetische Polymere, insbesondere Elastomere. Besonders bevorzugt sind Polystyrol oder Co- und Terpolymere von Styrol, z.B. solche, die in den obigen Punkten 4, 5 und 6 erwähnt sind. Ebenfalls bevorzugt sind Polyolefine, z.B. solche, die in den obigen Punkten 1 bis 3, insbesondere 1 und 2, erwähnt sind.

Die erfindungsgemässen Zusammensetzungen enthalten zweckmässigerweise 0,01 bis 10 %, bevorzugt 0.05 bis 5 %, insbesonders 0,1 bis 2 % einer Verbindung oder eines Gemisches von Verbindungen der Formel I, bezogen auf das Gesamtgewicht des zu stabilisierenden organischen Materials.

Neben Verbindungen der Formel I können die erfindungsgemässen Zusammensetzungen zusätlich herkömmliche Additive enthalten, wie beispielsweise

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-di-methylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert-butyl-3-methylphenol), 4,4′-Thio-bis-(6-tert.butyl-2-methylphenol).

1.4. Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.butylphenol), 4,4′-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3′-tert.butyl-4′-hydroxyphenyl)-butyrat], Bis-(3-tert.butyl-4-hydroxy-5-methylphenyl)-dicyclopentadien, Bis-[2-(3′-tert.butyl-2′-hydroxy-5′-methyl-benzyl)-6-tert.butyl-4-methyl-phenyl]-terephthalat.

1.5. Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)-dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphon säure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen

Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethylisocyanurat, N,N'-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.-amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3,-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenylundecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-di-methylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-disubstituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat,

Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Einarbeitung der Verbindungen dieser Erfindung sowie gegebenenfalls weiterer Additive in das organische Material erfolgt nach bekannten Methoden, beispielsweise vor oder während der Formgebung oder auch durch Aufbringen der gelösten oder dispergierten Verbindungen auf das organische Material, gegebenenfalls unter nachträglichem Verdunsten des Lösungsmittels. Die Verbindungen dieser Erfindung können auch in Form eines Masterbatches, der diese beispielsweise in einer Konzentration von 2,5 bis 25 Gew.-% enthält, den zu stabilisierenden Materialien zugesetzt werden.

Die Verbindungen dieser Erfindung können auch vor oder während der Polymerisation oder vor der Vernetzung zugegeben werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die Verbindungen dieser Erfindung können in Analogie zu bekannten Verfahren (z.B. US-A-4 507 420) hergestellt werden, beispielsweise durch Umsetzung eines Phenols der Formel II bzw. eines Gemisches aus Phenolen der Formeln II und IIa,

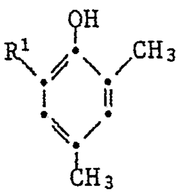 (II), 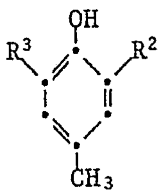 (IIa)

worin $R^1$, $R^2$ und $R^3$ die oben angegebenen Bedeutungen haben, mit Formaldehyd oder einem Formaldehydsyntheseequivalent, wie z.B. Dialkylacetal, insbesondere Dimethylacetal, in Gegenwart eines Katalysators bei einer Temperatur von 0 bis 100°C, bevorzugt 0 bis 60°C, und in einem organischen Lösungsmittel, wie beispielsweise in einem Chlorkohlenwasserstoff, wie z.B. Methylenchlorid, Chloroform oder Dichlorethan, oder in einem Ether, wie z.B. Tetrahydrofuran, Dimethoxyethan oder Dioxan, oder in Toluol, Essigsäureethylester oder in einem Alkohol, wie z.B. Ethanol oder Isopropanol, oder in einem Glykol oder Glykolmonoalkylether, oder auch in Wasser. Die Umsetzung kann gegebenenfalls in einer Stickstoffatmosphäre durchgeführt werden. Als geeignete Katalysatoren seien genannt: organische und anorganische, feste oder flüssige starke Säuren, wie z.B. Schwefelsäure, Phosphorsäure, Poly phosphorsäure, Salzsäure, p-Toluolsulfonsäure oder saure Ionenaustauscher, beispielsweise ®Nafion H. Der Katalysator kann gegebenenfalls auch in einer wässrigen Lösung vorliegen.

Wenn ein Gemisch aus den Phenolen II und IIa eingesetzt wird, kann das Molverhältnis dieser beiden Komponenten z.B. 1:5 bis 5:1 betragen; allerdings werden die Phenole II und IIa bevorzugt im stöchiometrischen Verhältnis, d.h. ca. 1:1 eingesetzt.

Das Molverhältnis Phenol II/Aldehyd bzw. Gemisch aus den Phenolen II und IIa/Aldehyd beträgt zweckmässigerweise 2:1 bis 2:2,5 und das Molverhältnis Phenol II/Katalysator bzw. Gemisch aus den Phenolen II und IIa/Katalysator 1:0,5 bis 1:2. Die Reaktionszeit kann z.B. 2 bis 20 Stunden betragen.

Es ist vorteilhaft, die Säure (Katalysator) nach der Umsetzung durch Waschen mit einer geeigneten Base bzw. mit Wasser zu entfernen.

Fällt das Produkt aus dem Reaktionsmedium aus, wird es zweckmässigerweise abfiltriert und direkt weiterverarbeitet; ansonsten kann es nach der Entfernung der Lösungsmittels nach üblichen Methoden (z.B. Umkristallisation oder Chromatographie) aufgearbeitet werden.

Als mögliche Varianten des oben beschriebenen Herstellungsverfahrens seien z.B. genannt:

1.) Phenol der Formel II bzw. Gemisch aus den Phenolen II und IIa, Formaldehyd, Katalysator und Lösungsmittel können bei 0°C gemischt und anschliessend bei einer Temperatur von 0 bis 100°C, bevorzugt 0 bis 60°C, bis zur erfolgten Umsetzung gerührt werden.

2.) Phenol der Formel II bzw. Gemisch aus den Phenolen II und IIa und Formaldehyd können in einem organischem Lösungsmittel vorgelegt werden und der Katalysator kann bei einer Temperatur von 0 bis 100°C, bevorzugt 0 bis 60°C, zugegeben werden.

3.) Phenol der Formel II bzw. Gemisch aus den Phenolen II und IIa und Katalysator können in einem Lösungsmittel vorgelegt werden und eine Formaldehydlösung kann bei 0 bis 100°C, bevorzugt 0 bis 60°C, zugegeben werden.

Fällt das Reaktionsprodukt als Gemisch von Verbindungen der Formel I an, so kann dieses, falls gewünscht, nach herkömmlichen Verfahren, z.B. chromatographischen Methoden, aufgetrennt werden.

Als weiteres mögliches Herstellungsverfahren der Verbindungen dieser Erfindung sei die Umsetzung von entweder einem Phenol der Formel II mit einer Verbindung der Formel III oder einem Phenol der Formel IIa mit einer Verbindung der Formel IIIa

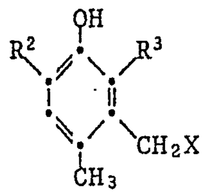 (III),

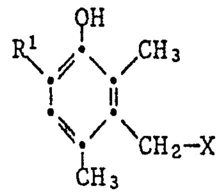 (IIIa),

worin $R^1$, $R^2$ und $R^3$ die oben angegebenen Bedeutungen besitzen und X z.B. Halogen, insbesondere Cl oder Br, Tosyl, Mesyl, Hydroxy, Alkoxy, bevorzugt $C_1$-$C_4$-Alkoxy, oder Acyloxy, bevorzugt $C_1$-$C_5$-Alkanoyloxy, ist, in Gegenwart eines Katalysators und in einem organischen Lösungsmittel genannt. Die Umsetzung kann z.B. bei einer Temperatur von 50°C erfolgen. Als Katalysator kann zweckmässigerweise eine Brönsted- oder Lewis-Säure, wie z.B. Zinkchlorid, verwendet werden. Beispiele für geeignete Lösungsmittel sind dieselben, wie in dem vorangehenden Herstellungsverfahren aufgeführt. Die Reaktionszeit kann z.B. 5 Stunden betragen.

Das molare Verhältnis der Verbindungen II/III bzw. IIa/IIIa beträgt beispielsweise 1:1 bis 1:2.

Die Ausgangsverbindungen sind bekannt, teilweise im Handel erhältlich und können ebenfalls in Analogie zu bekannten Verfahren hergestellt werden. Beispielsweise wird die Herstellung der Phenole der Formeln II und IIa in der DE-A-2 231 069 beschrieben.

Die folgenden Beispiele erläutern die Erfindung weiter. Alle Prozentangaben beziehen sich, ebenso wie in der übrigen Beschriebung auf das Gewicht, soweit nicht anders angegeben.

Beispiel 1: Herstellung von 3,3′-Dihydroxy-4,4′-dicyclohexyl-2,2′,6,6′-tetramethyl-diphenylmethan (Verbindung 1).

10,2 g 2,4-Dimethyl-6-cyclohexylphenol werden in 30 ml Chloroform gelöst und bei 0°C mit 7,3 g 96%iger Schwefelsäure versetzt. Dann werden in einer Stickstoffatmosphäre unter Rühren innerhalb von 15 Minuten 3,1 ml Formalin (36%ig) zugetropft.

Nach beendeter Zugabe wird das Reaktionsgemisch 2,5 Stunden unter Rückfluss gerührt. Anschliessend wird das Reaktionsgemisch abgekühlt und nach Zugabe von ca. 30 ml $CHCl_3$ wird die organische Phase mit Wasser neutral gewaschen, getrocknet und eingedampft.

Der Rückstand wird durch Umkristallisation mit Benzin gereinigt. Man erhält Kristalle mit einem Schmelzpunkt von 160-161°C.

| | | |
|---|---|---|
| Elementaranalyse: | Ber.(%) C = 82,81 | Gef.(%) C = 82,79 |
| | H = 9,59 | H = 9,79. |

Beispiel 2: Herstellung von 3,3′-Dihydroxy-4,4′-diethyl-2,2′6,6′-tetramethyl-diphenylmethan und 3,3′-Dihydroxy-4,2′-diethyl-2,4′,6,6′-tetramethyl-diphenylmethan (Verbindung 2).

Zu einem Gemisch aus 30 g 2,4-Dimethyl-6-ethylphenol, 216 ml $H_2SO_4$ (40%ig) und 90 ml Ethanol werden bei 80°C unter Rühren und in einer Stickstoffatmosphäre innerhalb von 1,5 Stunden 12,3 ml Formalin (36%ig) getropft. Das Reaktionsgemisch wird dann weitere 6 Stunden bei einer Temperatur von 80°C gehalten. Nach dem Abkühlen wird mit Ether extrahiert und die organische Phase mit Wasser neutral gewaschen.

Das Lösungsmittel wird abgedampft und nach Verreiben mit Petrolether erhält man ein kristallines Produkt mit einem Schmelzpunkt von 91-95°C.

| | | |
|---|---|---|
| Elementaranalyse: | Ber.(%) C = 80,73 | Gef.(%) C = 80,54 |
| | H = 9,03 | H = 9,19. |

$^1$H-NMR und GC-MS Analysen zeigen, dass das Produkt aus 2 Isomeren besteht [Gemisch aus 3,3′-Dihydroxy-4,4′-diethyl-2,2′,6,6′-tetramethyl-diphenylmethan und 3,3′-Dihydroxy-4,2′-diethyl-2,4′,6,6′-tetramethyl-diphenylmethan in Verhältnis 2:1].

Beispiel 3: Stabilisierung von Acrylnitril-Butandien-Styrol (ABS)

Die in Tabelle 1 angegebenen Additive werden in 40 ml eines geeigneten Lösungsmittelgemisches (Hexan-Isopropanol im Verhältnis 1:1) gelöst. Die Lösung wird unter kräftigem Rühren zu einer Dispersion von 100 g ABS in 600 g Wasser gegeben. Die Additiv-Lösung wird durch das ABS in kurzer Zeit (1 Minute) vollständig absorbiert. Das ABS-Pulver wird abgenutscht und 40 Stunden bei 40°C im Vakuum getrocknet. Dem trockenen Pulver werden 2 g Titandioxid (Pigment) sowie 1 g Ethylen-bis-stearinsäureamid (Gleitmittel) zugegeben. Die Mischung wird anschliessend 4 Minuten auf einem Zweiwalzenstuhl bei 180°C compoundiert.

Aus dem Walzfell wird bei 175°C eine Platte von 0,8 mm Dicke gepresst, aus welcher Prüflinge von 45 x 17 $mm^2$ gestanzt werden.

Die Prüfung auf Wirksamkeit des zugesetzten Additivs wird durch Hitzealterung in einem Umluftofen bei 180°C vorgenommen. Als Kriterium dient die Farbentwicklung nach 90 Minuten Prüfdauer. Die Farbintensität wird mit dem "Yellowness Index" nach ASTM D 1925 angegeben. Höhere Zahlen bedeuten intensiveren Gelbeindruck und damit stärkes Verfärbung durch Abbau.

Tabelle 1:

| Additiv | "Yellowness Index" nach 45 Minuten Ofenalterung |
|---|---|
| 0,5 % Dilaurylthiodipropionat | 80 |
| 0,25 % der Verbindung 1 und 0,5 % Dilaurylthiodipropionat | 24 |
| 0,25 % der Verbindung 2 und 0,5 % Dilaurylthiodipropionat | 27 |

**Patentansprüche**

1. Verbindungen der Formel I,

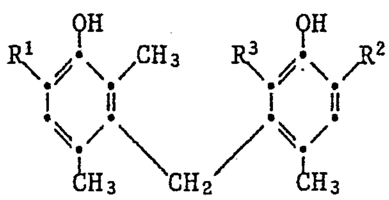

(I)

worin $R^1$ und $R^2$ unabhängig voneinander Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl sind und $R^3$ Methyl oder Ethyl bedeutet, mit der Bedingung, dass die Verbindung, 3,3'-Dihydroxy-2,2',4,4',6,6'-hexamethyldiphenylmethan ausgeschlossen ist.

2. Verbindungen gemäss Anspruch 1, worin $R^3$ Methyl bedeutet.

3. Verbindungen gemäss Anspruch 1 oder 2, worin $R^1$ und $R^2$ unabhängig voneinander Ethyl, Isopropyl, Isobutyl, am α-C-Atom verzweigtes $C_5$-$C_{20}$-Alkyl, unsubstituiertes oder durch $C_1$-$C_4$-ALkyl substituiertes $C_5$-$C_8$-Cycloalkyl, Benzyl oder Phenyl sind und $R^1$ zusätzlich Methyl bedeutet.

4. Verbindungen gemäss Anspruch 1 oder 2, worin $R^1$ und $R^2$ unabhängig voneinander Ethyl, Isopropyl oder $C_5$-$C_8$-Cycloalkyl sind.

5. Verbindungen gemäss Anspruch 1 oder 2, worin $R^1$ und $R^2$ unabhängig voneinander Ethyl, Isopropyl oder Cyclohexyl bedeuten.

6. Verbindungen gemäss Anspruch 1 oder 2, worin $R^1$ und $R^2$ Cyclohexyl sind.

7. Verbindungen gemäss Anspruch 1 oder 2, worin $R^1$ und $R^2$ gleich sind.

8. Verbindungen gemäss Anspruch 1, worin $R^1$ Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl ist, $R^2$ Methyl bedeutet und $R^3$ Ethyl darstellt.

9. Zusammensetzung ein gegen oxidativen, thermischen und/oder aktinischen Abbau empfindliches organisches Material und mindestens eine Verbindung gemäss Anspruch 1.

10. Zusammensetzung gemäss Anspruch 9, worin das organische Material ein synthetisches Polymer ist.

11. Zusammensetzung gemäss Anspruch 9, worin das organische Material ein Elastomer ist.

12. Zusammensetzung gemäss Anspruch 9, worin das organische Material Polystyrol oder ein Co- oder Terpolymeres von Styrol ist.

13. Zusammensetzung gemäss Anspruch 9, worin das organische Material ein Polyolefin ist.

14. Verwendung der Verbindungen gemäss Anspruch 1 zum Stabilisieren von organischem Material gegen oxidativen, thermischen und/oder aktinischen Abbau.

Patentansprüche für den folgenden Vertragsstaat: ES

1. Zusammensetzung enthaltend ein gegen oxidativen, thermischen und/oder aktinischen Abbau empfindliches organisches Material und mindestens eine Verbindung der Formel I,

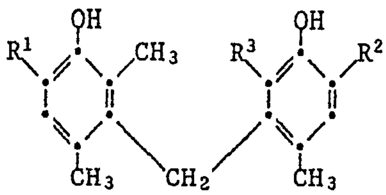 (I)

worin $R^1$ und $R^2$ unabhängig voneinander Methyl, Ethyl, Isopropyl, Isobutyl, am α-C-Atom verzweigtes $C_5$-$C_{20}$-Alkyl, unsubstituiertes oder durch $C_1$-$C_4$Alkyl substituiertes $C_5$-$C_8$-Cycloalkyl, Benzyl oder Phenyl sind und $R^3$ Methyl oder Ethyl bedeutet, mit der Bedingung, dass die Verbindung 3,3′-Dihydroxy-2,2′,4,4′,6,6′-hexamethyldiphenylmethan ausgeschlossen ist.

2. Zusammensetzung gemäss Anspruch 1, worin $R^3$ Methyl bedeutet.

3. Zusammensetzung gemäss Anspruch 1 oder 2, worin $R^1$ und $R^2$ unabhängig voneinander Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl sind und $R^1$ zusätzlich Methyl bedeutet.

4. Zusammensetzung gemäss Anspruch 1 oder 2, worin $R^1$ und $R^2$ unabhängig voneinander Ethyl, Isopropyl oder $C_5$-$C_8$-Cycloalkyl sind.

5. Zusammensetzung gemäss Anspruch 1 oder 2, worin $R^1$ und $R^2$ unabhängig voneinander Ethyl, Isopropyl oder Cyclohexyl bedeuten.

6. Zusammensetzung gemäss Anspruch 1 oder 2, worin $R^1$ und $R^2$ Cyclohexyl sind.

7. Zusammensetzung gemäss Anspruch 1 oder 2, worin $R^1$ und $R^2$ gleich sind.

8. Zusammensetzung gemäss Anspruch 1, worin $R^1$ Methyl, Ethyl, Isopropyl, Isobutyl, am α-C-Atom verzweigtes $C_5$-$C_{20}$-Alkyl, unsubstituiertes oder durch $C_1$-$C_4$-Alkyl substituiertes $C_5$-$C_8$-Cycloalkyl, Benzyl oder Phenyl ist, $R^2$ Methyl bedeutet und $R^3$ Ethyl darstellt.

9. Zusammensetzung gemäss Anspruch 1, worin das organische Material ein synthetisches Polymer ist.

10. Zusammensetzung gemäss Anspruch 1, worin das organische Material ein Elastomer ist.

11. Zusammensetzung gemäss Anspruch 1, worin das organische Material Polystyrol oder ein Co- oder Terpolymeres von Styrol ist.

12. Zusammensetzung gemäss Anspruch 1, worin das organische Material ein Polyolefin ist.

13. Verfahren zum Stabilisieren von organischem Material gegen oxidativen, thermischen und/oder aktinischen Abbau, dadurch gekennzeichnet, dass man dem organischen Material mindestens eine Verbindung der Formel I nach Anspruch 1 einverleibt.

14. Verfahren zum Herstellung von Verbindungen der Formel I gemäss Anspruch 1, dadurch gekennzeichnet, dass ein Phenol der Formel II oder ein Gemisch aus den Phenolen der Formeln II und IIa,

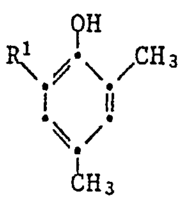 (II),

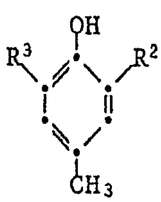 (IIa)

worin $R^1$, $R^2$ und $R^3$ die in Anspruch 1 angegebene Bedeutung haben, mit Formaldehyd oder einem Formaldehydsyntheseequivalent in Gegenwart eines Katalysators umgesetzt wird.

15. Verfahren zum Herstellung von Verbindungen der Formel I gemäss Anspruch 1, dadurch gekennzeichnet, dass entweder ein Phenol der Formel II mit einer Verbindung der Formel III oder ein Phenol der Formel IIa mit einer Verbindung der Formel IIIa,

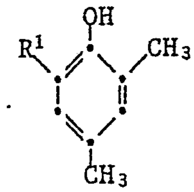 (II), 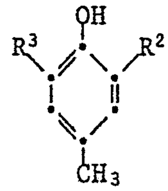 (IIa),

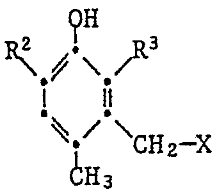 (III), 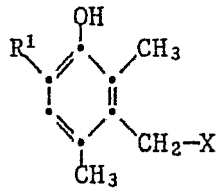 (IIIa)

wobei $R^1$, $R^2$ und $R^3$ die in Anspruch 1 angegebene Bedeutung haben und X Halogen, Tosyl, Mesyl, Hydroxy, Alkoxy oder Acyloxy ist, in Gegenwart eines Katalysators umgesetzt wird.